# EUROPÄISCHE PATENTSCHRIFT

(11) **EP 3 445 705 B1**
(45) Veröffentlichungstag und Bekanntmachung des Hinweises auf die Patenterteilung: **21.09.2022**
(21) Anmeldenummer: 17720387.4
(22) Anmeldetag: 18.04.2017
(51) Int. Cl.: B41J 2/175

(54) **VERFAHREN UND BEFÜLLUNGSVORRICHTUNG ZUM BEFÜLLEN EINES TRANSPORTBEHÄLTERS MIT EINEM FLUID**
METHOD AND FILLING DEVICE FOR FILLING A TRANSPORT CONTAINER WITH A FLUID
PROCÉDÉ ET DISPOSITIF DE REMPLISSAGE SERVANT À REMPLIR UN RÉSERVOIR DE TRANSPORT AVEC UN FLUIDE

(30) Priorität: 19.04.2016 DE 102016004612
(43) Veröffentlichungstag der Anmeldung: 27.02.2019
(73) Patentinhaber: Merck Patent GmbH, 64293 Darmstadt (DE)
(72) Erfinder: HAUKE, Guenter, 64367 Muehltal (DE); GARCIA DIEZ, Leticia, 64287 Darmstadt (DE); HILARIUS, Volker, 64823 Gross-Umstadt (DE)
(86) Internationale Anmeldenummer: PCT/EP2017/059093
(87) Internationale Veröffentlichungsnummer: WO 2017/182419

(56) Entgegenhaltungen:
- JP-A- 2006 321 677
- US-A- 4 804 464
- US-A1- 2003 205 285

## Beschreibung

Die Erfindung betrifft ein Verfahren zum Befüllen eines Transportbehälters mit einem Fluid, wobei das Fluid durch eine Reinigungsvorrichtung gefördert wird, bevor das Fluid in den Transportbehälter gefüllt wird.

In den letzten Jahren sind verschiedene organische Halbleitermaterialien entwickelt worden, die sich beispielsweise für die Herstellung von organischen Halbleiter-Bauteilen und insbesondere für die Herstellung von organischen Leuchtdioden und entsprechenden Displays eignen. Für die Verarbeitung der organischen Halbleitermaterialien eignen sich unter anderem verschiedene Drucktechnologien, mit denen die in einem geeigneten Lösungsmittel gelösten organischen Halbleitermaterialien auf eine vorgegebene Oberfläche aufgebracht werden können. Auf diese Weise können beispielsweise großflächige Displays, die aus einer sehr großen Anzahl von unabhängig voneinander ansteuerbaren organischen Leuchtdioden (OLED) aus organischen Halbleitermaterialien zusammengesetzt sind, mit aus der Praxis bekannten Tintenstrahl-Druckvorrichtungen gedruckt werden.

Die derzeit bekannten Drucktechnologien ermöglichen eine rasche und vom Verfahrensablauf her einfache Herstellung von Bauteilen und insbesondere von Displays aus organischen Halbleitermaterialien. Es hat sich allerdings gezeigt, dass für die Produktqualität der Bauteile und Displays eine nahezu unvermeidbare Verunreinigung der gelösten organischen Halbleitermaterialien mit Partikeln sowie gelösten Gasen von besonderer Bedeutung ist. Trotz großer Anstrengungen bei der Herstellung und dem Abfüllen von organischen Halbleitermaterialien können Verunreinigungen mit Fremdpartikeln kaum vermieden werden. Die in dem Lösungsmittel gelösten organischen Halbleitermaterialien weisen zudem eine hohe Empfindlichkeit gegenüber der Umgebungsluft und auch gegenüber Feuchtigkeit auf, so dass die organischen Halbleitermaterialien bereits nach einem kurzzeitigen Kontakt mit der Umgebungsluft eine produktgefährdende Menge von Gasen und/oder Feuchtigkeit aufnehmen können.

Um die für eine Weiterverarbeitung der organischen Halbleitermaterialien erforderliche Reinheit zu erreichen, werden die in einem geeigneten organischen Lösungsmittel gelösten Halbleitermaterialien üblicherweise in einem mehrstufigen Reinigungsprozess gereinigt, filtriert und entgast. Das gereinigte Fluid aus dem gelösten organischen Halbleitermaterial wird anschließend in Transportbehälter abgefüllt und von dem Herstellungsort der organischen Halbleitermaterialien zu einem Produktionsort für die jeweiligen Bauteile oder Displays gebracht, für deren Herstellung das organische Halbleitermaterial benötigt wird. Auch die Transportbehälter werden dabei vor dem Befüllen mit dem organischen Halbleitermaterial gereinigt, um die Verunreinigung des in den Transportbehältern abgefüllten und transportierten Fluids so gering wie möglich zu halten. Weiterhin wird auch an dem Produktionsort der jeweiligen Komponenten und Bauteile bei dem Anschließen des dorthin transportierten Transportbehälters an eine Produktionsvorrichtung sowie gegebenenfalls auch vor einer Inbetriebnahme sowie während des Betriebs der Produktionsvorrichtung ein erheblicher Aufwand betrieben, um eine Kontamination und Verunreinigung der die organischen Halbleitermaterialien enthaltenden Fluide so gering wie möglich zu halten.

Da die Materialkosten und der Herstellungsaufwand für viele organische Halbleitermaterialien sehr hoch sind, muss gleichzeitig versucht werden, das organische Halbleitermaterial möglichst effizient für die Produktherstellung zu verwenden und den Anteil des nicht für die Produktion der Komponenten verwendbaren Fluids eines einzelnen Transportbehälters möglichst zu minimieren. Beispielsweise darf durch die Reinigung des Fluids kein übermäßiger Anteil des Fluids für die anschließende Produktion verloren gehen. Zudem sollten Totvolumina von Anschlusseinrichtungen des Transportbehälters oder in den Vorrichtungen für das Reinigen des Fluids und das anschließende Befüllen des Transportbehälters möglichst klein sein, um den für die Produktion der Komponenten nicht nutzbaren Anteil des Fluids so gering wie möglich zu halten.

Da bereits kleinste Mengen von Verunreinigungen und gegebenenfalls einzelne Partikel einer Verunreinigung ein mit dem betreffenden organischen Halbleitermaterial hergestelltes Produkt wie beispielsweise ein großformatiges Display unbrauchbar machen können, werden oftmals sehr hohe Anforderungen an die Herstellung und den Transport des organischen Halbleitermaterials bis an den Produktionsort der betreffenden Komponenten bzw. Displays gestellt. In der Praxis werden deshalb üblicherweise während und nach der Herstellung der gelösten organischen Halbleitermaterialien stichprobenartige Messungen und Überprüfungen der Qualität des gelösten organischen Halbleitermaterials durchgeführt, um die vorgegebene Reinheit des Fluids nachweisen und gewährleisten zu können. Der damit einhergehende Aufwand für die Herstellung und Überprüfung des gelösten organischen Halbleitermaterials sowie für deren Transport bis an die jeweilige Produktionsstätte der betreffenden Produkte ist aufwändig und kostenintensiv.

US 4,804,464 offenbart eine Befüllungsvorrichtung gemäß dem Oberbegriff des Anspruchs 9.

Es wird deshalb als eine Aufgabe der vorliegenden Erfindung angesehen, ein Verfahren zum Befüllen eines Transportbehälters mit einem Fluid so auszugestalten, dass eine möglichst geringe Verunreinigung des Fluids in dem Transportbehälter mit einem möglichst geringen Aufwand erreicht werden kann.

Diese Aufgabe wird durch das Verfahren gemäß Anspruch 1 gelöst, wobei in einem Fluidreinigungsschritt das Fluid in einem Reinigungskreislauf mehrfach durch die Reinigungseinrichtung gefördert wird und mit einer Verunreinigungsmesseinrichtung eine Verunreinigungskenngröße einer Fluidprobenmenge in dem Reinigungskreislauf ermittelt wird, und dass ein Befüllungsvorgang des Transportbehälters mit dem Fluid erst beendet wird, nachdem die Verunreinigungskenngröße einen ersten Schwellenwert unterschreitet. In dem Reinigungskreislauf kann das Fluid mehrfach umgewälzt werden. Bei jedem Reinigungszyklus wird das Fluid durch die Reinigungseinrichtung gefördert und gereinigt. Die aus der Praxis bekannten Reinigungsverfahren wie beispielsweise Filtrieren oder Entgasen weisen prinzipbedingt und abhängig von der jeweiligen Umsetzung eine durchschnittliche bzw. maximale Reinigungseffizienz auf, so dass während eines Reinigungsschritts ein entsprechender Anteil von Verunreinigungen aus dem Fluid abgetrennt und entfernt werden kann. Erfahrungsgemäß kann in vielen Fällen nach einer einmaligen Reinigung des Fluids in einer Reinigungseinrichtung, die beispielsweise eine Filtereinrichtung oder eine Entgasungseinrichtung beinhaltet, noch keine ausreichende Reinheit des Fluids erreicht bzw. gewährleistet werden. Durch die Integration einer Verunreinigungsmesseinrichtung in den Reinigungskreislauf, in dem das Fluid mehrfach durch die Reinigungseinrichtung gefördert und dadurch kontinuierlich stärker gereinigt wird, kann die verbleibende Verunreinigung durch die Verunreinigungsmesseinrichtung jederzeit erfasst und für den weiteren Verfahrensablauf bzw. für das Befüllen des Transportbehälters mit dem gereinigten Fluid berücksichtigt werden. Dabei kann die Verunreinigungskenngröße kontinuierlich, in regelmäßigen oder vorgegebenen Zeitabständen oder aber nur bei Bedarf oder nach einer Anfrage eines Benutzers ermittelt werden. Der Aufwand für die Ermittlung der Verunreinigungskenngröße ist sehr gering.

Mit dem erfindungsgemäßen Verfahren kann der Verunreinigungsgehalt bzw. die Reinheit des in dem Transportbehälter gefüllten Fluids für jeden Transportbehälter ermittelt werden. Im Vergleich zu den aus dem Stand der Technik bekannten Verfahren, bei denen mit zusätzlichem Aufwand aus einzelnen bereits befüllten Transportbehältern gesonderte Fluidproben entnommen werden und diese Fluidproben auf Verunreinigungen untersucht werden, kann mit dem erfindungsgemäßen Verfahren ohne einen nennenswerten zusätzlichen Aufwand jeder einzelne Transportbehälter überprüft und der Verunreinigungsgehalt des darin abgefüllten Fluids kontrolliert werden.

Sollte während der Befüllung eines Transportbehälters festgestellt werden, dass die mit der Verunreinigungsmesseinrichtung ermittelte Verunreinigungskenngröße der Fluidprobenmenge, die der Verunreinigungsmesseinrichtung zugeführt wird, eine unerwünscht hohe Verunreinigung belegt, kann beispielsweise ein bereits in den Transportbehälter abgefüllter Anteil des Fluids wieder entnommen werden, um nachfolgend einen stärker gereinigten Fluidanteil wieder in den Transportbehälter einzufüllen. Der Befüllungsvorgang beinhaltet einzelne oder mehrfache Entnahmen einer bereits abgefüllten Fluidmenge aus dem Transportbehälter. Der Befüllungsvorgang für einen betreffenden Transportbehälter wird erst dann beendet, wenn die mit der Verunreinigungsmesseinrichtung gemessene Verunreinigungskenngröße einen Schwellenwert unterschreitet, der so vorgegeben ist, dass die gewünschte Reinheit des in dem Transportbehälter abgefüllten Fluids ausreichend zuverlässig erreicht und gewährleistet werden kann.

Das erfindungsgemäße Verfahren kann in vorteilhafter Weise für das Abfüllen verschiedener Fluide verwendet werden, für welche die Reinheit des abgefüllten Fluids bzw. eine möglichst geringe Verunreinigung des abgefüllten Fluids relevant sind. Ein Anwendungsbereich des erfindungsgemäßen Verfahrens betrifft organische Halbleitermaterialien, die in Lösung bzw. als Bestandteil eines flüssigen Tintenmaterials in einen Transportbehälter abgefüllt werden, um an dem vorgesehenen Verwendungsort wieder aus dem Transportbehälter entnommen und für die Herstellung eines organischen Halbleiter-Bauelements verwendet werden zu können. Dabei kann das organische Halbleitermaterial beispielsweise zur Herstellung von elektronischen oder optoelektronischen Vorrichtungen, wie zum Beispiel flüssig-beschichteten oder gedruckten elektronischen, optoelektronischen, photovoltaischen, sensorischen oder organischen elektrolumineszenten Vorrichtungen, vorzugsweise OLEDs und besonders bevorzugt von OLED-Displays verwendet werden. Es können jedoch auch andere Fluide in vorteilhafter Weise mit dem erfindungsgemäßen Befüllungsverfahren gereinigt und in Transportbehälter abgefüllt werden, die beispielsweise funktionale Komponenten oder gelöste Bestandteile enthalten, für deren Funktion oder Wirkung es nach deren Applikation notwendig ist, dass das Fluid vorgegebene Schwellenwerte für eine maximal zulässige Verunreinigung möglichst nicht überschreitet.

Einer Ausgestaltung des Erfindungsgedankens zufolge ist vorgesehen, dass mit dem Befüllen des Transportbehälters mit dem gereinigten Fluid aus dem Reinigungskreislauf erst begonnen wird, nachdem die Verunreinigungskenngröße einen zweiten Schwellenwert unterschreitet. Es ist in vielen Fällen zweckmäßig, dass das abzufüllende Fluid in dem Reinigungskreislauf zunächst mehrfach durch die Reinigungseinrichtung gefördert und dadurch mehrfach gereinigt wird, bevor mit dem Befüllen des Transportbehälters begonnen wird. Beispielsweise aus Gründen der Effizienz kann es jedoch ebenfalls zweckmäßig sein, dass ein geringer Anteil der in dem Reinigungskreislauf zirkulierenden Fluidmenge abgezweigt und in den Transportbehälter eingefüllt wird, obwohl eine vorgegebene Anzahl von vollständigen Durchströmungen des Reinigungskreislaufs noch nicht erreicht wurde. Der zweite Schwellenwert kann in diesem Fall früher als der erste Schwellenwert erreicht werden. Der Transportbehälter wird dann zunehmend mit einem immer stärker gereinigten Fluid befüllt, wobei die sich im Mittel einstellende Verunreinigung des gesamten Inhalts des Transportbehälters unterhalb eines vorgegebenen Schwellenwerts ist.

Es ist ebenfalls denkbar, dass der zweite Schwellenwert mit dem ersten Schwellenwert übereinstimmt und mit dem Befüllen des Transportbehälters erst begonnen wird, nachdem das in dem Reinigungskreislauf zirkulierende Fluid einen gewünschten Reinheitsgrad erreicht hat. In diesem Fall kann der Befüllungsvorgang ausschließlich über die bereits in den Transportbehälter abgefüllte Fluidmenge überwacht werden und der Befüllungsvorgang nach dem Abfüllen einer vorgegebenen Füllmenge des Fluids beendet werden.

Um die Zirkulation des Fluids in dem Reinigungskreislauf nicht durch eine für die Durchführung einer Messung notwendige Verweildauer des Fluids in der Verunreinigungsmesseinrichtung zu begrenzen, kann zweckmäßigerweise vorgesehen sein, dass die für die Ermittlung der Verunreinigungskenngröße vorgesehene Fluidprobenmenge aus dem Reinigungskreislauf abgezweigt, der Verunreinigungsmesseinrichtung zugeführt und nach der Ermittlung der Verunreinigungskenngröße wieder in den Reinigungskreislauf zurückgeführt wird. Dadurch kann das Fluid in dem Reinigungskreislauf mit einer hohen Strömungsrate zirkulieren, die gegebenenfalls durch eine von der Reinigungseinrichtung vorgegebene maximale Strömungsrate begrenzt ist. Die aus dem Reinigungskreislauf abgezweigte Fluidprobenmenge, die der Verunreinigungsmesseinrichtung zugeführt wird, kann dort unabhängig von der in dem Reinigungskreislauf vorgegebenen Strömungsrate verweilen, um ausreichend präzise und genaue Messungen zu ermöglichen. Dabei wird davon ausgegangen, dass das in dem Reinigungskreislauf zirkulierende Fluid ausreichend durchmischt und homogen ist, so dass die von der Fluidprobenmenge ermittelte Verunreinigungskenngröße charakteristisch für die Verunreinigung des in dem Reinigungskreislauf zirkulierenden Fluids ist.

Gemäß einer besonders vorteilhaften Ausgestaltung des Erfindungsgedankens ist vorgesehen, dass in einem Behälterreinigungsschritt eine vorab aus dem Reinigungskreislauf in den Transportbehälter eingebrachte Behälterfluidmenge wieder aus dem Transportbehälter entnommen und in den Reinigungskreislauf zurückgeführt wird. Es hat sich gezeigt, dass die Verunreinigung des Fluids in dem Transportbehälter nicht ausschließlich durch eine während der Herstellung des Fluids unvermeidbare Verunreinigung des Fluids bewirkt wird, sondern dass auch eine Verunreinigung des zunächst noch leeren, unbefüllten Transportbehälters einen merklichen Anteil zu der Verunreinigung des anschließend in den Transportbehälter befüllten Fluids beitragen kann. Eine gesonderte Reinigung des Transportbehälters ist aufwändig und kostenintensiv. Zudem könnte eine in den meisten Fällen unvermeidlich verbleibende Verunreinigung des leeren Transportbehälters nicht während des Befüllens des Transportbehälters erfasst und berücksichtigt werden. Aus diesem Grund kann der Transportbehälter in den Reinigungskreislauf eingebunden und von dem Fluid durchströmt werden, so dass Verunreinigungen in dem Transportbehälter von dem Fluid aufgenommen und während einer nachfolgenden Durchströmung der Reinigungseinrichtung in den Reinigungskreislauf herausgefiltert werden können.

Eine besonders zuverlässige Überwachung und Vorgabe der Reinheit des in den Transportbehälter abgefüllten Fluids kann dadurch erreicht werden, dass mit der Verunreinigungsmesseinrichtung eine Verunreinigungskenngröße der aus dem Transportbehälter entnommenen Behälterfluidmenge ermittelt wird und dass der Befüllungsvorgang des Transportbehälters mit dem Fluid erst beendet wird, nachdem die Verunreinigungskenngröße einen dritten Schwellenwert unterschreitet. Auf diese Weise kann sichergestellt werden, dass nicht nur das in den Transportbehälter eingefüllte Fluid, sondern auch das anschließend aus dem Transportbehälter entnommene Fluid eine vorgegebene Reinheit aufweist. Mit der in den Reinigungskreislauf integrierten Verunreinigungsmesseinrichtung kann dadurch nicht nur der Verunreinigungsgrad bzw. Reinheitsgrad des Fluids vor der Abfüllung in den Behälter, sondern auch der Reinheitsgrad des in dem Transportbehälter befindlichen Fluids nach dessen Entnahme erfasst bzw. überwacht und dadurch auch vorgegeben werden. Der nach der Entnahme des Fluids aus dem Transportbehälter ermittelte Reinheitsgrad entspricht in aller Regel auch dem Reinheitsgrad, den ein Anwender des Fluids beispielsweise bei der Herstellung von elektronischen Bauteilen oder Displays vorfindet, sofern während des Transports des Transportbehälters zu dem Anwender hin keine nachträgliche Verunreinigung des Fluids erfolgt. Dies kann durch eine geeignete Ausgestaltung des Transportbehälters weitestgehend verhindert werden.

Da erfahrungsgemäß eine Verunreinigung des Transportbehälters deutlich geringer als eine während der Herstellung des Fluids unvermeidbar aufgetretene Verunreinigung des Fluids selbst ist, ist erfindungsgemäß vorgesehen, dass der Behälterreinigungsschritt erst begonnen wird, nachdem eine in dem Fluidreinigungsschritt ermittelte Verunreinigungskenngröße des in dem Reinigungskreislauf geförderten Fluids einen vierten Schwellenwert unterschreitet. So kann beispielsweise das für die Befüllung des Transportbehälters vorgesehene Fluid zunächst in dem Reinigungskreislauf zirkulieren, bis eine Verunreinigungskenngröße auf ein Zehntel des ursprünglichen Werts abgesunken ist. Anschließend kann der Transportbehälter in den Reinigungskreislauf eingebunden und von dem zirkulierenden Fluid durchströmt werden, um die in dem Transportbehälter vorhandenen Verunreinigungen auszutragen. Das Fluid zirkuliert dabei weiterhin in dem Reinigungskreislauf, bis die Verunreinigungskenngröße auf ein Prozent des ursprünglichen Werts abgesunken ist und eine ausreichende Reinheit des in dem Reinigungskreislauf und durch den Transportbehälter hindurch zirkulierenden Fluids bestätigt wird.

Gemäß einer Ausgestaltung des Erfindungsgedankens ist vorgesehen, dass in dem Fluidreinigungsschritt das Fluid durch mindestens einen Partikelfilter und eine Entgasungseinrichtung gefördert wird. Eine Kombination eines Partikelfilters und einer Entgasungseinrichtung ist insbesondere bei dem Abfüllen von organischen Halbleitermaterialien zweckmäßig und vorteilhaft, deren anschließende Nutzung sowohl durch Partikelverunreinigungen als auch durch gasförmige Verunreinigungen beeinträchtigt und eingeschränkt werden kann. Es ist ebenfalls denkbar, dass mehrere Partikelfilter mit übereinstimmenden Filtereigenschaften miteinander kombiniert werden, um die Effizienz der Reinigungseinrichtung zu steigern. Es können auch mehrere Partikelfilter mit unterschiedlichen Filtereigenschaften bzw. verschiedenen Filterklassen kombiniert und beispielsweise zwei oder drei Partikelfilter nacheinander angeordnet werden, die zunehmend kleinere Partikeldurchmesser herausfiltern können. Auch eine Kombination mehrerer Entgasungseinrichtungen kann zweckdienlich sein, um beispielsweise unterschiedliche Gase herauszufiltern oder um die Effizienz der Entgasung bei einem Durchgang durch die Reinigungseinrichtung zu steigern.

Zweckmäßigerweise ist erfindungsgemäß vorgesehen, dass sich die Verunreinigungskenngröße aus einer Partikelgehaltskenngröße und aus einer Gasgehaltskenngröße zusammensetzt, die jeweils mit der Verunreinigungsmesseinrichtung erfasst werden. So können Verunreinigungen durch Partikel und durch einen Gasgehalt unabhängig voneinander kontrolliert und über geeignete Schwellenwerte für den Ablauf und die Steuerung des erfindungsgemäßen Verfahrens herangezogen und berücksichtigt werden. Es ist ebenfalls möglich, dass mehrere Partikelgehaltskenngrößen gleichzeitig erfasst und für den Verfahrensablauf berücksichtigt werden, so dass beispielsweise für unterschiedliche Bereiche von Partikeldurchmessern der jeweilige Partikelgehalt des abzufüllenden Fluids überwacht und die Reinigung des Fluids fortgesetzt wird, bis in allen relevanten Bereichen der Partikeldurchmesser die jeweils vorgegebenen Schwellenwerte unterschritten oder aber erreicht bzw. eingehalten werden.

Die Erfindung betrifft auch eine Befüllungsvorrichtung zum Befüllen eines Transportbehälters mit einem Fluid gemäß Anspruch 9, wobei die Vorrichtung einen aus Fluidleitungsabschnitten gebildeten Reinigungskreislauf aufweist und in dem Reinigungskreislauf eine Reinigungseinrichtung und eine Verunreinigungsmesseinrichtung angeordnet sind, und dass in dem Reinigungskreislauf eine Verzweigung mit einer Behälterbefüllungsleitung angeordnet ist, die zum Befüllen an den Transportbehälter angeschlossen werden kann. Mit der erfindungsgemäßen Befüllungsvorrichtung kann in einfacher Weise das für die Befüllung des Transportbehälters vorgesehene Fluid in einem Reinigungskreislauf umgewälzt und dadurch mehrfach durch die in dem Reinigungskreislauf angeordnete Reinigungseinrichtung geführt werden. Gleichzeitig kann mit der Verunreinigungsmesseinrichtung eine bereits erreichte Reinigungswirkung kontrolliert werden. Nach einer ausreichenden Aufreinigung des Fluids in dem Reinigungskreislauf kann der Transportbehälter, der über eine Verzweigung mit dem Reinigungskreislauf verbunden ist, mit dem gereinigten Fluid befüllt werden.

Erfindungsgemäß ist vorgesehen, dass in dem Reinigungskreislauf eine Einmündung mit einer Behälterrückleitung angeordnet ist, die zum Entleeren des Transportbehälters an den Transportbehälter angeschlossen werden kann. Der Transportbehälter kann auf diese Weise in den Reinigungskreislauf mit eingebunden werden, so dass das in dem Reinigungskreislauf zirkulierende Fluid auch durch den Transportbehälter hindurchgeführt werden kann. Dadurch können in dem Transportbehälter befindliche Verunreinigungen von dem Fluid aufgenommen und aus dem Transportbehälter abgeführt werden. Auf diese Weise kann ohne nennenswerten zusätzlichen Aufwand eine zusätzliche Reinigung des Transportbehälters vorgenommen werden, um eine Verunreinigung des für eine spätere Verwendung in den Transportbehälter abgefüllten Fluids zu vermeiden.

Der Transportbehälter kann dabei entweder vollständig in den Reinigungskreislauf eingebunden und von der gesamten in dem Reinigungskreislauf umgewälzten Fluidmenge durchströmt werden. Es ist ebenfalls denkbar, dass der Transportbehälter über eine Bypass-Leitung an den Reinigungskreislauf angeschlossen wird und lediglich von einer vorgegebenen Teilmenge des in dem Reinigungskreislaufs zirkulierenden Fluids durchströmt wird.

In vorteilhafter Weise kann der Reinigungskreislauf eine Vorratsanschlusseinrichtung aufweisen, mit der ein Vorratsbehälter für das Fluid an den Reinigungskreislauf angeschlossen werden kann. Der Reinigungskreislauf kann so ausgelegt sein, dass die gesamte in einem Vorratsbehälter vorgegebene Fluidmenge kontinuierlich durch den Reinigungskreislauf zirkulieren kann. Bei Bedarf können dann nacheinander oder gegebenenfalls gleichzeitig mehrere Transportbehälter an den Reinigungskreislauf angeschlossen und mit dem gereinigten Fluid befüllt werden. Es ist ebenfalls möglich, dass lediglich die für die Befüllung des Transportbehälters vorgesehene Fluidmenge in den Reinigungskreislauf eingespeist wird und dort aufgereinigt wird, um eine möglichst rasche Aufreinigung der für die Befüllung des Transportbehälters vorgesehenen Fluidmenge zu ermöglichen.

Zweckmäßiger Weise weist die Reinigungseinrichtung mindestens einen Partikelfilter und eine Entgasungseinrichtung auf. In vielen Fällen kann es vorteilhaft sein, dass in Strömungsrichtung mindestens ein erster Partikelfilter vor und mindestens ein zweiter Partikelfilter nach der Entgasungseinrichtung angeordnet ist. Es ist ebenfalls denkbar, dass mehrere Partikelfilter mit einer übereinstimmenden Filterwirkung oder aber mit in Strömungsrichtung kleiner werdenden Maschenweiten oder Porendurchmessern kombiniert werden. In gleicher Weise können auch mehrere gleichartige oder verschiedene Entgasungseinrichtungen miteinander kombiniert, beziehungsweise im Wechsel mit Partikelfiltern eingesetzt werden.

Die Verunreinigungsmesseinrichtung ist zweckmäßiger Weise in Strömungsrichtung nach der Reinigungseinrichtung angeordnet, so dass der durch die Reinigungseinrichtung bewirkte Reinigungseffekt von der Verunreinigungsmesseinrichtung bereits erfasst werden kann.

Um auch die gegebenenfalls durch einen angeschlossenen und mit dem Fluid durchströmten Transportbehälter verursachten Verunreinigungen erfassen und für die weitere Verfahrenssteuerung berücksichtigen zu können ist vorgesehen, dass die Einmündung der Behälterrückleitung in Strömungsrichtung vor der Verunreinigungsmesseinrichtung angeordnet ist.

In Abhängigkeit von den im Einzelfall verwendeten Messmethoden und Messvorrichtungen der Verunreinigungsmesseinrichtung kann es erfindungsgemäß vorteilhaft sein, dass in dem Reinigungskreislauf ein Bypassleitungsabschnitt angeordnet ist, in dem das Fluid durch die Verunreinigungsmesseinrichtung gefördert werden kann, so dass lediglich eine vorgebbare Fluidprobenmenge durch die Verunreinigungsmesseinrichtung gefördert wird. In vielen Fällen ist die für eine Erfassung einer Verunreinigungskenngröße erforderliche Messdauer erheblich größer als die Zeitdauer, die das Fluid benötigt, um durch die Reinigungseinrichtung hindurch zu strömen und dabei gereinigt zu werden. Um einen möglichst großen Durchsatz und eine rasche Aufreinigung des in dem Reinigungskreislaufs zirkulierenden Fluids zu ermöglichen kann es deshalb zweckmäßig sein, dass lediglich eine kleine Fluidprobenmenge in der Verunreinigungsmesseinrichtung überprüft und ausgewertet wird, während ein überwiegender Anteil des zirkulierenden Fluids an der Verunreinigungsmesseinrichtung vorbeigefördert und bereits erneut der Reinigungseinrichtung zugeführt werden kann.

Nachfolgend werden exemplarisch Ausführungsbeispiele des Erfindungsgedankens näher erläutert, die schematisch in der Zeichnung dargestellt sind. Es zeigt:
Figur 1 eine schematische Darstellung einer erfindungsgemäßen Befüllungsvorrichtung mit einem Reinigungskreislauf, mit einer in dem Reinigungskreislauf angeordneten Reinigungseinrichtung und mit einer Verunreinigungsmesseinrichtung, sowie mit einer Verzweigung mit einer Behälterbefüllungsleitung, und
Figur 2 eine schematische Darstellung einer abweichend ausgestalteten Befüllungsvorrichtung.

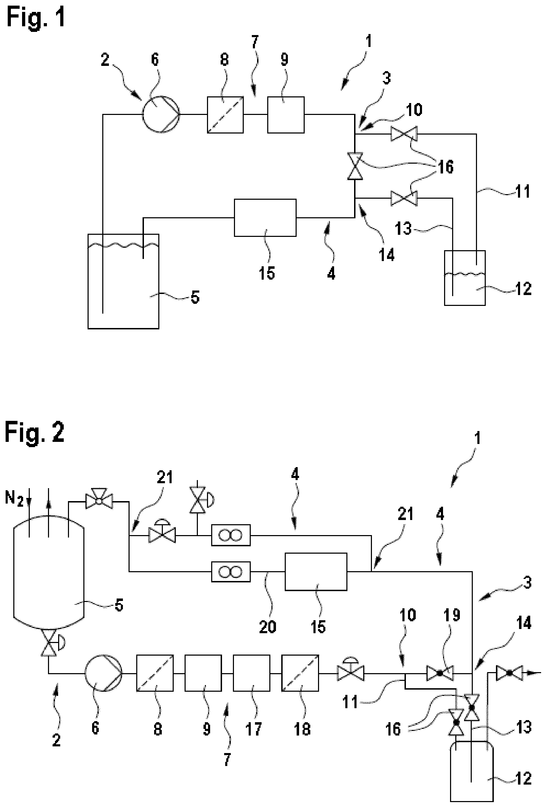

Eine in Figur 1 exemplarisch dargestellte Befüllungsvorrichtung weist einen Reinigungskreislauf 1 auf, der aus mehreren Fluidleitungsabschnitten 2, 3, 4 zusammengesetzt ist. Der Fluidleitungsabschnitt 2 ist an einem beispielsweise 10 Liter oder 30 Liter fassenden Vorratsbehälter 5 so angeschlossen, dass in dem Vorratsbehälter 5 befindliches Fluid mit einer Pumpeinrichtung 6 aus dem Vorratsbehälter 5 zu einer Reinigungseinrichtung 7 gefördert werden kann. Die Reinigungseinrichtung 7 weist einen Partikelfilter 8, beispielsweise einen Membranfilter mit einem Porendurchmesser von 1 µm auf. In Strömungsrichtung nach dem Partikelfilter 8 ist eine Entgasungseinrichtung 9 angeordnet, mit welcher der Gasgehalt in dem Fluid reduziert werden kann.

An den Fluidleitungsabschnitt 2 schließt der Fluidleitungsabschnitt 3 an. In dem Fluidleitungsabschnitt 3 ist eine erste Verzweigung 10 vorgesehen, die mit einer Behälterbefüllungsleitung 11 verbunden ist. Die Behälterbefüllungsleitung 11 ist lösbar mit einem für die Befüllung vorgesehenen Transportbehälter 12 verbunden. Der Transportbehälter 12 kann ein Fassungsvermögen von beispielsweise 200 ml oder von einem Liter aufweisen und je nach Verwendungszweck als Liefergebinde oder Druckerkartusche ausgestaltet sein. Eine Behälterrückleitung 13 führt von dem Transportbehälter 12 zurück zu dem Fluidleitungsabschnitt 3, so dass das Fluid aus dem Transportbehälter 12 über eine zweite Verzweigung oder eine Einmündung 14 zurück in den Fluidleitungsabschnitt 3 und damit zurück in den Reinigungskreislauf 1 für das Fluid gefördert werden kann.

Anschließend wird das Fluid durch den Fluidleitungsabschnitt 4 geführt, in welchem eine Verunreinigungsmesseinrichtung 15 angeordnet ist. Mit der Verunreinigungsmesseinrichtung 15 kann eine Verunreinigungskenngröße für das durchströmende Fluid ermittelt werden. Der Fluidleitungsabschnitt 4 mündet wieder in den Vorratsbehälter 5, wodurch der Reinigungskreislauf 1 geschlossen wird.

Es sind erfindungsgemäß verschiedene Verfahrensabläufe möglich, um aus dem Vorratsbehälter 5 eine Fluidmenge zu entnehmen und nach einer ausreichenden Aufreinigung in dem Reinigungskreislauf 1 in den Transportbehälter 12 abzufüllen.

Das Fluid kann in dem Reinigungskreislauf 1 zirkulieren und kontinuierlich in der Reinigungseinrichtung 7 zunehmend gereinigt werden, bis eine mit der Verunreinigungsmesseinrichtung 15 ermittelte Verunreinigungskenngröße einen ersten Schwellenwert für den maximal zulässigen Verunreinigungsgehalt unterschreitet. Anschließend kann der Transportbehälter 12 mit dem gereinigten Fluid befüllt und von der Befüllungsvorrichtung getrennt werden.

Das Fluid kann auch zunächst in dem Reinigungskreislauf 1 zirkulieren, ohne dass der Transportbehälter 12 angeschlossen ist und von dem Fluid durchströmt wird. Mit der Verunreinigungsmesseinrichtung 15 wird kontinuierlich eine Verunreinigungskenngröße ermittelt und das Fluid in dem Reinigungskreislauf 1 umgewälzt und zirkuliert, bis ein vorgegebener zweiter Schwellenwert für den Verunreinigungsgehalt erreicht bzw. unterschritten wird. Anschließend wird durch Umschalten von Ventilen 16 der Transportbehälter 12 in den Reinigungskreislauf 1 einbezogen und von dem bereits gereinigten Fluid durchströmt. Dabei werden eventuell in dem Transportbehälter 12 befindliche Verunreinigungen von dem Fluid aufgenommen, zunächst in der Verunreinigungsmesseinrichtung 15 erfasst und in nachfolgenden Durchströmungen des Fluids durch die Reinigungseinrichtung 7 herausgefiltert. Die Zirkulation des Fluids durch den Transportbehälter 12 kann fortgesetzt werden, bis die in der Verunreinigungsmesseinrichtung 15 ermittelte Verunreinigungskenngröße einen dritten Schwellenwert unterschreitet. Der dritte Schwellenwert kann dem ersten Schwellenwert entsprechen, der in dem vorangehend erörterten Ausführungsbeispiel genannt und verwendet wurde. Es kann auch ein hiervon abweichender Schwellenwert vorgegeben werden, um nach der Reinigung des Transportbehälters 12 beispielsweise einen weniger strengen Verunreinigungsgehalt durch die Verunreinigungsmesseinrichtung 15 vorzugeben, da eine Kontamination durch den bereits gereinigten Transportbehälter 12 ausgeschlossen werden kann.

Es ist ebenfalls möglich, dass der Transportbehälter 12 bereits ab der ersten Zirkulation des Fluids durch den Reinigungskreislauf 1 mit eingebunden ist und von dem Fluid durchströmt wird.

In allen Fällen kann erreicht werden, dass für die tatsächlich in den Transportbehälter 12 abgefüllte Fluidmenge deren Verunreinigungen kontrolliert und unterhalb eines vorgegebenen Schwellenwerts reduziert wurden, bevor der Befüllungsvorgang beendet und der befüllte Transportbehälter 12 von der Befüllungsvorrichtung getrennt und entnommen wird. Eine nachfolgende Kontrollmessung ist nicht mehr erforderlich.

In Fig. 2 ist exemplarisch eine abweichend ausgestaltete Befüllungsvorrichtung schematisch dargestellt. Die Reinigungseinrichtung 7 weist zusätzlich zu der Entgasungseinrichtung 9 eine Sauerstoffsonde 17 auf. Der erste Partikelfilter 8 ist in Strömungsrichtung vor der Entgasungseinrichtung 9 und der Sauerstoffsonde 17 angeordnet. Ein zweiter Partikelfilter 18 ist in Strömungsrichtung nach der Sauerstoffsonde 17 angeordnet. Der erste Partikelfilter 8 weist einen Membranfilter mit einem Porendurchmesser von 0,1 µm auf. Der zweite Partikelfilter 18 weist einen Membranfilter mit einem Porendurchmesser von 0,05 µm auf.

Der Transportbehälter 12 ist ebenfalls über Ventile 16 in den Reinigungskreislauf 1 eingebunden. Über weitere Ventile 19 sind zusätzliche Steuerungsmöglichkeiten für die Fluidzirkulation gegeben.

Die Verunreinigungsmesseinrichtung 15 ist in einem Bypassleitungsabschnitt 20 angeordnet, der über Verzweigungen 21 mit dem Fluidleitungsabschnitt 4 verbunden ist. Durch die Verunreinigungsmesseinrichtung 15 strömt jeweils nur eine kleine Fluidprobenmenge, die lediglich einen geringen Anteil des in dem Reinigungskreislauf 1 zirkulierenden Fluids darstellt. Über Durchflussmesseinrichtungen 22 kann der jeweilige Anteil der durch den Bypassleitungsabschnitt 20 und durch den parallel geführten Fluidleitungsabschnitt 4 durchströmenden Fluidmenge erfasst, bzw. kontrolliert werden.

Unabhängig von der jeweiligen Ausgestaltung der Befüllungsvorrichtung bzw. von den in Fig. 1 und in Fig. 2 gezeigten Ausführungsbeispielen ist es möglich, dass an Stelle eines starren Transportbehälters 12, der beispielsweise eine Flasche oder ein metallischer Behälter sein kann, ein flexibler Transportbehälter verwendet wird, der beispielsweise ein Beutel oder ein flexibler Kunststoffbehälter sein kann. Wenn der Transportbehälter 12 in den Reinigungskreislauf 1 mit einbezogen wird, kann bei dem starren Transportbehälter 12 über entsprechende Lanzen oder Druckventile eine nahezu beliebige Fluidmenge entnommen oder nachgefüllt werden, wobei ständig ein wechselnder Anteil des Fluids in dem Transportbehälter 12 verbleibt. Insbesondere bei einem flexiblen Transportbehälter kann es auch zweckdienlich sein, dass jeweils aufeinanderfolgend der flexible Transportbehälter vollständig befüllt und anschließend vollständig entleert wird.

Nachfolgend werden beispielhaft einige exemplarisch mit der erfindungsgemäßen Befüllungsvorrichtung durchgeführte Befüllungsvorgänge beschrieben.

Das Fluid beinhaltet organische Halbleitermaterialien, bzw. OLED-Materialien und gegebenenfalls weitere Zusatzstoffe. Die Komponenten des Fluids werden gemäß einer Einwaagetabelle in einen Behälter mit dem vorgelegten Lösungsmittel- bzw. Lösungsmittelgemisch gegeben. Danach werden die Materialien in einem Taumelmischer gemischt und aufgelöst.

Bei den in den Ausführungsbeispielen eingesetzten Fluiden handelt es sich um kommerzielle Produkte der Merck KGaA.

### Auführungsbeispiele

### Beispiel 1:

Das gemischte Fluid wird mittels Gasdruck mit gereinigtem Stickstoff in den Vorratsbehälter 5 einer Befüllungsvorrichtung gefördert, die im Wesentlichen der in Fig. 2 gezeigten Befüllungsvorrichtung entspricht. Der zur Förderung verwendete Stickstoff wurde vorher über einen Gasfilter filtriert. Der Vorratsbehälter 5 ist gemäß Fig. 1 verbunden mit einer Pumpe 6 (Levitronix), einem Membran-Entgaser 9 und zwei PTFE-Filtern 8, 18 mit einer Porengröße von jeweils 0,1 µm und 0,05 µm (Entegris). Der Reinigungsprozess erfolgt erfindungsgemäß in der Weise, dass das Fluid zunächst über die Pumpe 6, die Filtereinrichtung 7 und die Verunreinigungsmesseinrichtung 15, einem optischen Partikelzähler, zyklisiert und die gemessene Verunreinigungskenngröße mit gewünschten bzw. vorgegebenen Zielparametern verglichen wird. Nach dem Passieren der Verunreinigungsmesseinrichtung 15 wird das Fluid (Tinte bzw. auch nur das Lösungsmittel) in den Vorratsbehälter 5 zurückgeführt und mit dem dort befindlichen Fluid vermischt. Das Fluid wird solange in dem Reinigungskreislauf umgewälzt, bis der gewünschte Reinigungseffekt erreicht und mit der Verunreinigungsmesseinrichtung 15 nachgewiesen wird.

Die nachfolgend wiedergegebenen Tabellen 1 und 2 zeigen eine Zusammenfassung der mit der Verunreinigungsmesseinrichtung 15 gemessenen Partikelgehalte für Partikel unterschiedlicher Größe für Lösungsmittel und Tinten für verschiedene Zyklisierungszeiten. Die jeweiligen Fluide brauchen unterschiedliche Reinigungsdauern, bis der Zielwert erreicht wird. Während das Reserver Cleaning Solvent und die MRE Tinten eine Zyklisierungszeit von 30 Minuten bis eine Stunde brauchen, benötigen die anderen Fluide etwa 2 bis 6 Stunden, um den vorgegebenen Zielwert zu erreichen, bzw. die jeweiligen Schwellenwerte für einen Verunreinigungsgehalt zu unterschreiten.

**Tabelle 1: Partikelgehalt für verschiedene Fluide nach zunehmenden Zyklisierungszeiten. Partikelzahlen pro 10 ml Lösung.**

| Produkt Name | Größe | 1 min | 10 min | 30 min | 1 std | 2 std | 6 std | 10 std | 15 std | 20 std |
|---|---|---|---|---|---|---|---|---|---|---|
| MRE3-0669 | >0,15 µm | 14 | 1 | 0 | | | | | | |
| | >0,2 µm | 4 | 0 | 0 | | | | | | |
| | >0,3 µm | 2 | 0 | 0 | | | | | | |
| | >0,4 µm | 2 | 0 | 0 | | | | | | |
| | >0,5 µm | 2 | 0 | 0 | | | | | | |
| Reserver Cleaning Solvent 1 | >0,15 µm | 848 | 139 | 8 | 5 | 0 | | | | |
| | >0,2 µm | 451 | 74 | 4 | 3 | 0 | | | | |
| | >0,3 µm | 104 | 24 | 1 | 1 | 0 | | | | |
| | >0,4 µm | 68 | 18 | 1 | 1 | 0 | | | | |
| | >0,5 µm | 42 | 12 | 0 | 1 | 0 | | | | |
| MRE3-7306 | >0,15 µm | 9 | 4 | 3 | 3 | 0 | 1 | | | |
| | >0,2 µm | 1 | 1 | 0 | 1 | 0 | 0 | | | |
| | >0,3 µm | 0 | 0 | 0 | 0 | 0 | 0 | | | |
| | >0,4 µm | 0 | 0 | 0 | 0 | 0 | 0 | | | |
| | >0,5 µm | 0 | 0 | 0 | 0 | 0 | 0 | | | |
| MHL3-6689 | >0,2 µm | 614 | 82 | 38 | 20 | 10 | 7 | 6 | 11 | |
| | >0,3 µm | 31 | 6 | 0 | 0 | 2 | 0 | 0 | 0 | |
| | >0,4 µm | 18 | 4 | 0 | 0 | 2 | 0 | 0 | 0 | |
| | >0,5 µm | 14 | 3 | 0 | 0 | 2 | 0 | 0 | 0 | |
| MHL3-8873 | >0,2 µm | 537 | 158 | 93 | 45 | 30 | 25 | 22 | 15 | |
| | >0,3 µm | 27 | 3 | 4 | 0 | 1 | 0 | 1 | 1 | |
| | >0,4 µm | 12 | 1 | 2 | 0 | 0 | 0 | 0 | 1 | |
| | >0,5 µm | 8 | 1 | 0 | 0 | 0 | 0 | 0 | 1 | |

**Tabelle 2: Partikelgehalt für verschiedene Fluide nach zunehmenden Zyklisierungszeiten. Partikelzahlen pro 10 ml Lösung.**

| Produkt Name | Größe | 1 min | 10 min | 30 min | 1 std | 2 std | 6 std | 10 std | 15 std | 20 std |
|---|---|---|---|---|---|---|---|---|---|---|
| MGE3-2449 | >0,15 µm | 188 | 22 | 28 | 8 | 2 | 12 | 5 | 0 | |
| | >0,2 µm | 106 | 8 | 8 | 4 | 1 | 1 | 1 | 0 | |
| | >0,3 µm | 44 | 3 | 3 | 2 | 0 | 0 | 0 | 0 | |
| | >0,4 µm | 36 | 1 | 2 | 1 | 0 | 0 | 0 | 0 | |
| | >0,5 µm | 27 | 0 | 1 | 1 | 0 | 0 | 0 | 0 | |
| MGE3-5237 | >0,15 µm | 205 | 36 | 11 | 6 | 2 | 4 | 4 | 1 | |
| | >0,2 µm | 50 | 11 | 1 | 0 | 1 | 1 | 1 | 0 | |
| | >0,3 µm | 5 | 5 | 0 | 0 | 0 | 1 | 0 | 0 | |
| | >0,4 µm | 2 | 5 | 0 | 0 | 0 | 1 | 0 | 0 | |
| | >0,5 µm | 1 | 4 | 0 | 0 | 0 | 0 | 0 | 0 | |
| MHL3-2892 | >0,15 µm | 436 | 114 | 43 | 17 | 15 | 8 | 11 | 14 | 7 |
| | >0,2 µm | 84 | 23 | 6 | 2 | 1 | 0 | 3 | 2 | 1 |
| | >0,3 µm | 16 | 8 | 2 | 1 | 0 | 0 | 0 | 0 | 0 |
| | >0,4 µm | 10 | 4 | 1 | 1 | 0 | 0 | 0 | 0 | 0 |
| | >0,5 µm | 8 | 3 | 0 | 1 | 0 | 0 | 0 | 0 | 0 |
| MBL3-6282 | >0,2 µm | 37 | 35 | 51 | 66 | 48 | 16 | 15 | 5 | 3 |
| | >0,3 µm | 9 | 6 | 17 | 29 | 11 | 0 | 0 | 1 | 0 |
| | >0,4 µm | 6 | 6 | 11 | 19 | 4 | 0 | 0 | 1 | 0 |
| | >0,5 µm | 3 | 2 | 7 | 13 | 3 | 0 | 0 | 1 | 0 |
| MHL3-9183 | >0,15 µm | 893 | 499 | 269 | 136 | 97 | 64 | 50 | 59 | 49 |
| | >0,2 µm | 163 | 89 | 40 | 23 | 15 | 10 | 6 | 5 | 6 |
| | >0,3 µm | 11 | 16 | 3 | 3 | 2 | 0 | 0 | 0 | 0 |
| | >0,4 µm | 5 | 11 | 1 | 1 | 0 | 0 | 0 | 0 | 0 |
| | >0,5 µm | 2 | 8 | 1 | 1 | 0 | 0 | 0 | 0 | 0 |

### Beispiel 2:

Nach dem das betreffende Fluid gemäß Beipiel 1 in dem Reinigungskreislauf aufgereinigt vorliegt wird der Abfüllprozess gestartet. Dabei wird der Transportbehälter 12 in den Reinigungskreislauf 1 einbezogen. Die aus dem Transportbehälter 12 wieder entnommene Fluidmenge wird dann über das Ventil 16 und die Verzweigung 14 wieder in den Reinigungszyklus zurückgeführt. In der Rückleitung zu dem Vorratsbehälter 5 befinden sich eine Durchflussmesseinrichtung und die Verunreinigungsmesseinrichtung 15, mit welcher der Gehalt an gelösten Gasen insgesamt und speziell der Gehalt an Sauerstoff erfasst werden kann. Der Transportbehälter 12 wird so lange mit bereits gereinigtem Fluid gespült, bis die Zielparameter in dem Rückfluss aus dem Transportbehälter 12 erreicht werden. Als Beispiel wurden mehrere 150 ml Flaschen (gereinigte Merck Braunglasflaschen) als Transportbehälter 12 mit dem Fluid "Reserver Cleaning Solvent" abgefüllt. Dabei wurden die Flaschen in den Reinigungskreislauf 1 solange einbezogen, bis der Zielparameter erreicht ist. In der Tabelle 3 sind die Partikelmessung für die unterschiedlichen Partikelgrößen für die Flaschen F1, F2, F3 und F6 zusammengefasst. Während bei der Flasche F1, F2 und F6 zwischen 1 und 2 Stunden nötig sind bis der Zielwert erreicht ist, braucht die Flasche F3 nur 10 Minuten.

**Tabelle 3: Befüllung des Fluids "Reserver Cleaning solvent" in 150 ml Braunglasflaschen. Partikelzahlen pro 10 ml Lösung.**

| Flasche Nr | Spülung Zyklus | Größe | 1 min | 10 min | 30 min | 40 min | 50 min | 1 Std | 2 std |
|---|---|---|---|---|---|---|---|---|---|
| F1 | 1 | >0,15 µm | 848 | 139 | 8 | 3 | 1 | 5 | 1 |
| | | >0,2 µm | 451 | 74 | 4 | 2 | 1 | 3 | 0 |
| | | >0,3 µm | 104 | 24 | 1 | 2 | 1 | 1 | 0 |
| | | >0,4 µm | 68 | 18 | 1 | 1 | 0 | 1 | 0 |
| | | >0,5 µm | 42 | 12 | 0 | 0 | 0 | 1 | 0 |
| F1 | 2 | >0,15 µm | 2 | 7 | 11 | 3 | 3 | 0 | |
| | | >0,2 µm | 1 | 3 | 7 | 2 | 2 | 0 | |
| | | >0,3 µm | 1 | 3 | 5 | 1 | 2 | 0 | |
| | | >0,4 µm | 1 | 0 | 4 | 0 | 1 | 0 | |
| | | >0,5 µm | 1 | 0 | 1 | 0 | 1 | 0 | |
| F2 | 1 | >0,15 µm | 185 | 2 | 1 | 0 | 1 | 0 | |
| | | >0,2 µm | 78 | 0 | 1 | 0 | 1 | 0 | |
| | | >0,3 µm | 13 | 0 | 0 | 0 | 1 | 0 | |
| | | >0,4 µm | 9 | 0 | 0 | 0 | 0 | 0 | |
| | | >0,5 µm | 8 | 0 | 0 | 0 | 0 | 0 | |
| F3 | 1 | >0,15 µm | 4 | 0 | | | | | |
| | | >0,2 µm | 2 | 0 | | | | | |
| | | >0,3 µm | 0 | 0 | | | | | |
| | | >0,4 µm | 0 | 0 | | | | | |
| | | >0,5 µm | 0 | 0 | | | | | |
| F6 | 1 | >0,15 µm | 679 | 27 | 18 | 8 | 7 | 5 | |
| | | >0,2 µm | 298 | 15 | 10 | 6 | 4 | 0 | |
| | | >0,3 µm | 71 | 4 | 3 | 4 | 1 | 0 | |
| | | >0,4 µm | 38 | 2 | 3 | 4 | 0 | 0 | |
| | | >0,5 µm | 18 | 1 | 2 | 3 | 0 | 0 | |

### Beispiel 3:

Nach dem das gewünschte Fluid gemäß Beispiel 1 im Reinigungskreislauf aufgereinigt vorliegt, wird der Abfüllprozess gestartet. In diesem Beispiel wird die Befüllung in Transportbehälter 12 beschrieben, die als Druckerkartuschen ausgebildet sind. Dafür beinhaltet die Befüllungsvorrichtung die zusätzliche Funktion der Spülung der Transportbehälter 12 durch Befüllung und Entleerung mit dem bereits gereinigten Fluid im Wechsel. Dieser wechselnde Betrieb wird mehrmals wiederholt, bis die gewünschte Verunreinigungskonzentration erreicht und ein vorgegebener Schwellenwert für die Verunreinigungskenngröße unterschritten wird. Dazu wird während der Entleerung des Transportbehälters 12 die Behälterrückleitung 13 mit dem Fluidleitungsabschnitt 3 und nachfolgend mit der Verunreinigungsmesseinrichtung 15 zur Bestimmung der Verunreinigungskenngröße verbunden. Nach der Passage durch die Verunreinigungsmesseinrichtung 15 wird das Fluid wieder in den Vorratsbehälter 5 zurückgeführt und mit dem dort befindlichen Fluid wieder vermischt.

Das vorher gemäß Beispiel 1 gereinigte Produkt MBL3-6282 wurde in zwei Druckerkartuschen abgefüllt. Diese Druckerkartuschen wurden mehreren Flushingzyklen unterzogen die jeweils aus einem Befüllen und anschließenden Entleeren des Transportbehälters 12 bestehen. In der Tabelle 4 sind die Daten zum Gehalt an Partikeln der unterschiedlichen Größen für die Druckerkartuschen 1 und 2 dargestellt. Während bei der Druckerkartusche 1 vier Flushingszyklen nötig sind um den gewünschten Wert zu erreichen, sind die Zielwerte bei der Druckerkartusche 2 bereits nach nur zwei Zyklen erreicht.

**Tabelle 4: Befüllung von MBL3-6282 in zwei Druckerkartuschen. Partikelzahlen pro 10 ml Lösung.**

| Flushing Nr. | Größe | **Druckerkartusche Nr** | |
|---|---|---|---|
| | | **1** | **2** |
| Flushing 1 | >0,2 µm | 1138 | 583 |
| | >0,3 µm | 253 | 71 |
| | >0,4 µm | 185 | 46 |
| | >0,5 µm | 139 | 33 |
| Flushing 2 | >0,2 µm | 281 | 282 |
| | >0,3 µm | 41 | 20 |
| | >0,4 µm | 29 | 12 |
| | >0,5 µm | 21 | 8 |
| Flushing 3 | >0,2 µm | 161 | |
| | >0,3 µm | 36 | |
| | >0,4 µm | 29 | |
| | >0,5 µm | 22 | |
| Flushing 4 | >0,2 µm | 229 | |
| | >0,3 µm | 18 | |
| | >0,4 µm | 12 | |
| | >0,5 µm | 9 | |

## Patentansprüche

1. Verfahren zum Befüllen eines Transportbehälters (12) mit einem Fluid, wobei das Fluid durch eine Reinigungseinrichtung (7) gefördert wird, bevor das Fluid in den Transportbehälter (12) gefüllt wird, wobei in einem Fluidreinigungsschritt das Fluid in einem Reinigungskreislauf (1) mehrfach durch die Reinigungseinrichtung (7) gefördert wird und mit einer Verunreinigungsmesseinrichtung (15) eine Verunreinigungskenngröße einer Fluidprobenmenge in dem Reinigungskreislauf (1) ermittelt wird, dass der Befüllungsvorgang einzelne oder mehrere Entnahmen einer bereits abgefüllten Fluidmenge aus dem Transportbehälter (12) beinhaltet, und dass ein Befüllungsvorgang des Transportbehälters (12) mit dem Fluid erst beendet wird, nachdem die Verunreinigungskenngröße einen ersten Schwellenwert unterschreitet, und der so vorgegeben ist, dass die gewünschte Reinheit des in dem Transportbehälter (12) abgefüllten Fluids ausreichend zuverlässig erreicht und gewährleistet wird.

2. Verfahren nach Anspruch 1, **dadurch gekennzeichnet, dass** mit dem Befüllen des Transportbehälters (12) mit dem gereinigten Fluid aus dem Reinigungskreislauf (1) erst begonnen wird, nachdem die Verunreinigungskenngröße einen zweiten Schwellenwert unterschreitet.

3. Verfahren nach Anspruch 1 oder Anspruch 2, **dadurch gekennzeichnet, dass** die für die Ermittlung der Verunreinigungskenngröße vorgesehene Fluidprobenmenge aus dem Reinigungskreislauf (1) abgezweigt, der Verunreinigungsmesseinrichtung (15) zugeführt und nach der Ermittlung der Verunreinigungskenngröße wieder in den Reinigungskreislauf (1) zurückgeführt wird.

4. Verfahren nach einem der vorangehenden Ansprüche, **dadurch gekennzeichnet, dass** in einem Behälterreinigungsschritt eine vorab aus dem Reinigungskreislauf (1) in den Transportbehälter (12) eingebrachte Behälterfluidmenge wieder aus dem Transportbehälter (12) entnommen und in den Reinigungskreislauf (1) zurückgeführt wird.

5. Verfahren nach Anspruch 4, **dadurch gekennzeichnet, dass** mit der Verunreinigungsmesseinrichtung (15) eine Verunreinigungskenngröße der aus dem Transportbehälter (12) entnommenen Behälterfluidmenge ermittelt wird und dass der Befüllungsvorgang des Transportbehälters (12) mit dem Fluid erst beendet wird, nachdem die Verunreinigungskenngröße einen dritten Schwellenwert unterschreitet.

6. Verfahren nach Anspruch 4 oder Anspruch 5, **dadurch gekennzeichnet, dass** der Behälterreinigungsschritt erst begonnen wird, nachdem eine in dem Fluidreinigungsschritt ermittelte Verunreinigungskenngröße des in dem Reinigungskreislauf (1) geförderten Fluids einen vierten Schwellenwert unterschreitet.

7. Verfahren nach einem der vorangehenden Ansprüche, **dadurch gekennzeichnet, dass** in dem Fluidreinigungsschritt das Fluid durch mindestens einen Partikelfilter (8) und durch eine Entgasungseinrichtung (9) gefördert wird.

8. Verfahren nach einem der vorangehenden Ansprüche, **dadurch gekennzeichnet, dass** sich die Verunreinigungskenngröße aus einer Partikelgehaltskenngröße und aus einer Gasgehaltskenngröße zusammensetzt, die jeweils mit der Verunreinigungsmesseinrichtung (15) erfasst werden.

9. Befüllungsvorrichtung zum Befüllen eines Transportbehälters (12) mit einem Fluid, wobei die Vorrichtung einen aus Fluidleitungsabschnitten (2, 3, 4) gebildeten Reinigungskreislauf (1) aufweist und in dem Reinigungskreislauf (1) eine Reinigungseinrichtung (7) und eine Verunreinigungsmesseinrichtung (15) angeordnet sind, und dass in dem Reinigungskreislauf (1) eine Verzweigung (10) mit einer Behälterbefüllungsleitung angeordnet ist, die zum Befüllen an den Transportbehälter (12) angeschlossen werden kann, **dadurch gekennzeichnet, dass** in dem Reinigungskreislauf (1) eine Einmündung (14) mit einer Behälterrückleitung (13) angeordnet ist, die zum Entleeren des Transportbehälters (12) an den Transportbehälter (12) angeschlossen werden kann, so dass der Transportbehälter (12) in den Reinigungskreislauf mit eingebunden und das in dem Reinigungskreislauf zirkulierende Fluid auch durch den Transportbehälter (12) hindurchgeführt wird.

10. Befüllungsvorrichtung nach Anspruch 9, **dadurch gekennzeichnet, dass** der Reinigungskreislauf (1) eine Vorratsan-schlusseinrichtung aufweist, mit der ein Vorratsbehälter (5) für das Fluid an den Reinigungskreislauf (1) angeschlossen werden kann.

11. Befüllungsvorrichtung nach Anspruch 9 oder 10, **dadurch gekennzeichnet, dass** die Reinigungseinrichtung (7) mindestens einen Partikelfilter (8) und eine Entgasungseinrichtung (9) aufweist.

12. Befüllungsvorrichtung nach Anspruch 11, **dadurch gekennzeichnet, dass** in Strömungsrichtung mindestens ein erster Partikelfilter (8) vor und mindestens ein zweiter Partikelfilter (18) nach der Entgasungseinrichtung (9) angeordnet ist.

13. Befüllungsvorrichtung nach einem der Ansprüche 9 bis 12, **dadurch gekennzeichnet, dass** die Verunreinigungsmesseinrichtung (15) in Strömungsrichtung nach der Reinigungseinrichtung (7) angeordnet ist.

14. Befüllungsvorrichtung nach einem der Ansprüche 9 bis 13, **dadurch gekennzeichnet, dass** die Einmündung (14) mit der Behälterrückleitung (13) in Strömungsrichtung vor der Verunreinigungsmesseinrichtung (15) angeordnet ist.

15. Befüllungsvorrichtung nach einem der Ansprüche 9 bis 14, **dadurch gekennzeichnet, dass** in dem Reinigungskreislauf (1) ein Bypassleitungsabschnitt (20) angeordnet ist, durch den das Fluid durch die Verunreinigungsmesseinrichtung (15) gefördert werden kann, so dass lediglich eine vorgebbare Fluidprobenmenge durch die Verunreinigungsmesseinrichtung (15) gefördert wird.

## Claims

1. Method for filling a transport container (12) with a fluid, where the fluid is conveyed through a purification device (7) before the fluid is introduced into the transport container (12), where, in a fluid purification step, the fluid is conveyed through the purification device (7) a number of times in a purification circuit (1) and a contamination factor of an amount of fluid sample in the purification circuit (1) is determined by means of a contamination measurement device (15), where the filling operation includes the single or multiple removal from the transport container (12) of an amount of fluid that has already been introduced, and where a filling operation of the transport container (12) with the fluid is only terminated after the contamination factor has fallen below a first threshold value, and which is specified so that the desired purity of the fluid introduced into the transport container (12) is achieved and ensured sufficiently reliably.

2. Method according to Claim 1, **characterised in that** the filling of the transport container (12) with the purified fluid from the purification circuit (1) is only commenced after the contamination factor has fallen below a second threshold value.

3. Method according to Claim 1 or Claim 2, **characterised in that** the amount of fluid sample intended for the determination of the contamination factor is diverted off from the purification circuit (1), fed to the contamination measurement device (15) and, after the determination of the contamination factor, fed back into the purification circuit (1) again.

4. Method according to one of the preceding claims, **characterised in that**, in a container cleaning step, an amount of container fluid that has previously been introduced into the transport container (12) from the purification circuit (1) is removed from the transport container (12) again and fed back into the purification circuit (1).

5. Method according to Claim 4, **characterised in that** a contamination factor of the amount of container fluid that has been removed from the transport container (12) is determined by means of the contamination measurement device (15) and **in that** the filling operation of the transport container (12) with the fluid is only terminated after the contamination factor has fallen below a third threshold value.

6. Method according to Claim 4 or Claim 5, **characterised in that** the container cleaning step is only commenced after a contamination factor, determined in the fluid purification step, of the fluid conveyed in the purification circuit (1) has fallen below a fourth threshold value.

7. Method according to one of the preceding claims, **characterised in that**, in the fluid purification step, the fluid is conveyed through at least one particle filter (8) and through a degassing device (9).

8. Method according to one of the preceding claims, **characterised in that** the contamination factor is composed of a particle content factor and a gas content factor, each of which is determined by means of the contamination measurement device (15).

9. Filling device for filling a transport container (12) with a fluid, where the device has a purification circuit (1) formed from fluid line sections (2, 3, 4), and a purification device (7) and a contamination measurement device (15) are arranged in the purification circuit (1), and where a branch (10) with a container filling line, which can be connected to the transport container (12) for filling, is arranged in the purification circuit (1), **characterised in that** a junction (14) with a container return line (13), which can be connected to the transport container (12) for emptying of the transport container (12), is arranged in the purification circuit (1), so that the transport container (12) is integrated into the purification circuit and the fluid circulating in the purification circuit is also passed through the transport container (12).

10. Filling device according to Claim 9, **characterised in that** the purification circuit (1) has a stock connection device, by means of which a stock container (5) for the fluid can be connected to the purification circuit (1).

11. Filling device according to Claim 9 or 10, **characterised in that** the purification device (7) has at least one particle filter (8) and a degassing device (9).

12. Filling device according to Claim 11, **characterised in that** at least one first particle filter (8) is arranged upstream of the degassing device (9) and at least one second particle filter (18) is arranged downstream of the degassing device (9).

13. Filling device according to one of Claims 9 to 12, **characterised in that** the contamination measurement device (15) is arranged downstream of the purification device (7).

14. Filling device according to one of Claims 9 to 13, **characterised in that** the junction (14) with the container return line (13) is arranged upstream of the contamination measurement device (15).

15. Filling device according to one of Claims 9 to 14, **characterised in that** a bypass line section (20), through which the fluid can be conveyed through the contamination measurement device (15) in such a way that only a predefinable amount of fluid sample is conveyed through the contamination measurement device (15), is arranged in the purification circuit (1).

## Revendications

1. Procédé pour remplir un moyen de contenance de transport (12) avec un fluide, dans lequel le fluide est convoyé au travers d'un dispositif de purification (7) avant que le fluide ne soit introduit à l'intérieur du moyen de contenance de transport (12), dans lequel, au niveau d'une étape de purification de fluide, le fluide est convoyé au travers du dispositif de purification (7) un certain nombre de fois dans un circuit de purification (1) et un facteur de contamination d'une quantité d'échantillon de fluide dans le circuit de purification (1) est déterminé au moyen d'un dispositif de mesure de contamination (15), dans lequel l'opération de remplissage inclut le prélèvement une seule fois ou de multiples fois, à partir du moyen de contenance de transport (12), d'une quantité de fluide qui a déjà été introduite, et dans lequel une opération de remplissage du moyen de contenance de transport (12) avec le fluide est seulement terminée après que le facteur de contamination a chuté en deçà d'une première valeur de seuil, laquelle première valeur de seuil est spécifiée de telle sorte que la pureté souhaitée du fluide qui est introduit à l'intérieur du moyen de contenance de transport (12) soit réalisée et assurée de façon suffisamment fiable.

2. Procédé selon la revendication 1, **caractérisé en ce que** le remplissage du moyen de contenance de transport (12) avec le fluide purifié en provenance du circuit de purification (1) est seulement commencé après que le facteur de contamination a chuté en deçà d'une deuxième valeur de seuil.

3. Procédé selon la revendication 1 ou la revendication 2, **caractérisé en ce que** la quantité d'échantillon de fluide visée pour la détermination du facteur de contamination est dérivée à partir du circuit de purification (1), est alimentée sur le dispositif de mesure de contamination (15) et, après la détermination du facteur de contamination, est retournée à nouveau à l'intérieur du circuit de purification (1).

4. Procédé selon l'une quelconque des revendications précédentes, **caractérisé en ce que**, lors d'une étape de nettoyage de moyen de contenance, une quantité de fluide de moyen de contenance qui a été introduite au préalable à l'intérieur du moyen de contenance de transport (12) depuis le circuit de purification (1) est prélevée à partir du moyen de contenance de transport (12) à nouveau et est retournée à l'intérieur du circuit de purification (1).

5. Procédé selon la revendication 4, **caractérisé en ce qu'**un facteur de contamination de la quantité de fluide de moyen de contenance qui a été prélevée à partir du moyen de contenance de transport (12) est déterminé au moyen du dispositif de mesure de contamination (15) et **en ce que** l'opération de remplissage du moyen de contenance de transport (12) avec le fluide est seulement terminée après que le facteur de contamination a chuté en deçà d'une troisième valeur de seuil.

6. Procédé selon la revendication 4 ou la revendication 5, **caractérisé en ce que** l'étape de nettoyage de moyen de contenance est seulement commencée après qu'un facteur de contamination, qui est déterminé au niveau de l'étape de purification de fluide, du fluide qui est convoyé dans le circuit de purification (1) a chuté en deçà d'une quatrième valeur de seuil.

7. Procédé selon l'une quelconque des revendications précédentes, **caractérisé en ce que**, au niveau de l'étape de purification de fluide, le fluide est convoyé au travers d'au moins un filtre à particules (8) et au travers d'un dispositif de dégazage (9).

8. Procédé selon l'une quelconque des revendications précédentes, **caractérisé en ce que** le facteur de contamination est composé d'un facteur de teneur en particules et d'un facteur de teneur en gaz dont chacun est déterminé au moyen du dispositif de mesure de contamination (15).

9. Dispositif de remplissage pour remplir un moyen de contenance de transport (12) avec un fluide, dans lequel le dispositif comporte un circuit de purification (1) qui est formé à partir de sections de ligne de fluide (2, 3, 4), et un dispositif de purification (7) et un dispositif de mesure de contamination (15) sont agencés dans le circuit de purification (1), et dans lequel un branchement (10) avec une ligne de remplissage de moyen de contenance, qui peut être connectée au moyen de contenance de transport (12) pour le remplissage, est agencé dans le circuit de purification (1), **caractérisé en ce qu'**une jonction (14) avec une ligne de retour de moyen de contenance (13), qui peut être connectée au moyen de contenance de transport (12) pour le vidage du moyen de contenance de transport (12), est agencée dans le circuit de purification (1), de telle sorte que le moyen de contenance de transport (12) soit intégré à l'intérieur du circuit de purification et que le fluide qui circule dans le circuit de purification soit également passé au travers du moyen de contenance de transport (12).

10. Dispositif de remplissage selon la revendication 9, **caractérisé en ce que** le circuit de purification (1) comporte un dispositif de connexion de fluide en réserve au moyen duquel un moyen de contenance de fluide en réserve (5) pour le fluide peut être connecté au circuit de purification (1).

11. Dispositif de remplissage selon la revendication 9 ou 10, **caractérisé en ce que** le dispositif de purification (7) comporte au moins un filtre à particules (8) et un dispositif de dégazage (9).

12. Dispositif de remplissage selon la revendication 11, **caractérisé en ce qu'**au moins un premier filtre à particules (8) est agencé en amont du dispositif de dégazage (9) et au moins un second filtre à particules (18) est agencé en aval du dispositif de dégazage (9).

13. Dispositif de remplissage selon l'une quelconque des revendications 9 à 12, **caractérisé en ce que** le dispositif de mesure de contamination (15) est agencé en aval du dispositif de purification (7).

14. Dispositif de remplissage selon l'une quelconque des revendications 9 à 13, **caractérisé en ce que** la jonction (14) avec la ligne de retour de moyen de contenance (13) est agencée en amont du dispositif de mesure de contamination (15).

15. Dispositif de remplissage selon l'une quelconque des revendications 9 à 14, **caractérisé en ce qu'**une section de ligne de dérivation (20), au travers de laquelle le fluide peut être convoyé au travers du dispositif de mesure de contamination (15) de telle sorte que seulement une quantité pouvant être prédéfinie d'échantillon de fluide soit convoyée au travers du dispositif de mesure de contamination (15), est agencée dans le circuit de purification (1).
